# EUROPEAN PATENT SPECIFICATION

(11) **EP 2 255 677 B1**
(45) Date of publication and mention of the grant of the patent: **02.04.2014**
(21) Application number: 10164238.7
(22) Date of filing: 28.05.2010
(51) Int. Cl.: A23L 1/00, A23L 1/236, A61K 9/20

(54) **Suclarose formulation and production process thereof**
Suclaroseformulierung und Produktionsverfahren dafür
Formulation de suclarose et son procédé de production

(30) Priority: 24.06.2009 TR 200904862; 29.05.2009 TR 200904205
(43) Date of publication of application: 01.12.2010
(73) Proprietor: MONTERO GIDA SANAYI VE TICARET A.S., Istinye, Istanbul 34460 (TR)
(72) Inventor: Toksöz, Ahmet, 34398 Istanbul (TR); Cifter, Ümit, Maslak Istanbul, TR-34398, (TR); Türkyilmaz, Ali, 34398 Istanbul (TR); Portakalkökü, Pinar, 34398 Istanbul (TR); Celik, Devrim, 34398 Istanbul (TR)
(74) Representative: Sevinç, Erkan

(56) References cited:
- EP-A- 1 629 730
- WO-A2-2010/081722
- CN-A- 101 543 480
- US-A1- 2002 122 823
- US-A1- 2003 216 461
- US-A1- 2004 191 400
- US-A1- 2010 143 486
- US-B1- 6 399 591
- US-B1- 7 390 503
- PARMAR, J.; RANE M.: "Tablet formulation design and manufacture : oral immediate release application", PHARMA TIMES, vol. 41, no. 4, April 2009 (2009-04), XP002558515,
- Ajinomoto AminoScience: "L-leucine", , 2 December 2008 (2008-12-02), XP002653227, Retrieved from the Internet: URL:http://web.archive.org/web/20081202061 009/http://www.ajiaminoscience.com/Product s/ProductDetails.aspx?MaterialNo=30394 [retrieved on 2011-07-25]
- RÖSCHEISEN ET AL: "Preparation and optimization of -leucine as lubricant for effervescent tablet formulations", PHARMACEUTICA ACTA HELVETIAE, vol. 70, no. 2, 1 July 1995 (1995-07-01), pages 133-139, XP55003578, ISSN: 0031-6865, DOI: 10.1016/0031-6865(95)00006-U
- LACHMAN, L.; SCHWARTZ, J.B.: "Pharmaceutical dosage forms: tablets", January 1989 (1989-01), MARCEL DEKKER, INC., NEW YORK, USA, XP002558516, * page 189 *

## Description

### Field of Invention

The present invention relates to newly-developed sucralose formulations and to the production process thereof. The present invention more particularly relates to a sucralose formulation, having improved dissolution and solubility rates and not enhancing the glycemic index, as well as to the production process of said sucralose formulation.

### Prior Art

Sucralose, a compound with the chemical name 1,6-dichloro-1,6-dideoxy-beta-D-fructofuranosyl-4-chloro-4-deoxy alpha-D-galactopyranoside, has the following chemical structure.

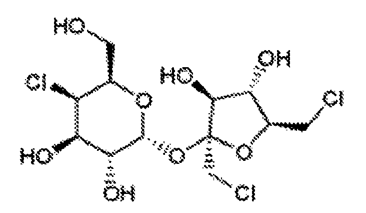

As it has been reported in the relevant scientific literature, sucralose has a sweetening effect which is around 300 to 1000 times stronger than that of sugar. (Handbook of Pharmaceutical Excipients - 5th Edition, p. 742)

The patent application GB1543167 discloses the use of sucralose molecule as a sweetener. The patent application W02004086885 describes a formulation, which makes use of lactose as a filling agent or filler. Lactose is reported in many resources to have a glycemic index of around 46. This fact is unfavorable for the diabetics.

US7390503 B1 discloses an ondansetron solid orally disintegrating dosage form for oral administration having at least one first water-dispersible component or water-insoluble cellulose derivative, a component having a -CHOH functional group, a disintegrating agent and at least one lubricant is provided. The dosage form can comprise ondansetron, a hydrophilic polymer such as microcrystalline cellulose, a component having a -CHOH functional group such as mannitol or xylitol and a disintegrating agent such as crospovidone. The lubricant may be a mixture of magnesium stearate, sodium stearyl fumarate and colloidal silicon dioxide. The invention provides a non-effervescent tablet comprising the ondansetron dosage form.

PARMAR, J.; RANE M.: "Tablet formulation design and manufacture: oral immediate release application", PHARMA TIMES, vol. 41, no 4, April 2009, generally disclose various excipients with respect to manufacture of tablet forms.

EP1629730 A1 discloses a functional food ingredient, which replaces sugar on a 1/1 weight and/or volume basis in food recipes containing sucrose, with a substantial caloric reduction in view. The functional food replacement for sucrose according to the invention comprises prebiotic fibres and sweeteners, and possibly other non selective fibres, minerals, vitamins and probiotic strains.

Ajinomoto Aminoscience : " L-leucine" of 02.12.2008 discloses leucine that can be used as a flavoring substance and as a lubricant for tablet production.

RÖSCHEISEN ET AL: "Preparation and optimization of leucine as lubricant for effervescent tablet formulations" PHARMACEUTICA ACTA HELVETIAE disclose spray dried and milled L-leucine was compared as lubricants for effervescent tablet formulations with regard to their lubrication properties, disintegration time, and crushing strength.

The patent application EP0267809 aims to provide protection for a concentrated mixture which is composed of sucralose and oligosaccharide. The glycemic index of maltodextrin used in this mixture is higher than 100. This is also an unfavorable case for the diabetics who use sweeteners. The patent application EP0313234 describes a synergistic mixture composed of sucralose and oligosaccharide. The patent application EP00390438 discloses a mixture of isomaltulose and sucralose. Isomaltulose here, albeit present at lower rates, has an affect that enhances the glycemic index.

The patent application WO2008036233 describes sweetener formulations. The auxiliary agents disclosed there embraces sugar alcohols. Mannitol, among said agents, leads to flowability-related problems during production, particularly sticking onto the surfaces of punches used for tablet manufacture, therefore rendering the production problematic. On the other hand, many sweetener formulations do not dissolve at acceptable rates in beverages in which they are used, causing to unwanted circumstances with respect to users. In result, the art of sweetener products requires a novelty to provide the advantages of not increasing the glycemic index, of simple production, and of satisfactory dissolution rates.

### Object and Brief Description of Invention

The present invention relates to a sweetener formulation, eliminating all aforesaid problems and brining additional advantages to the relevant prior art.

An object of the present invention is to eliminate the problems related to decreasing flowability and to the sticking of materials to production apparatuses.

A further object of the present invention is to obtain a sweetener formulation with improved dissolution rates particularly when used in beverages.

A novelty is realized in sweetener formulations to carry out all objects, referred to above and to emerge from the following detailed description.

According to a preferred embodiment of the present invention, this novelty is realized with sucralose in an amount of 1-15% as a high intensity sweetener, croscarmellose sodium and colloidal silicone dioxide with a weight ratio between 20:1 and 1:10 (croscarmellose sodium : colloidal silicone dioxide) sodium stearyl fumarate, and with at least one filler.

According to a preferred embodiment, the present invention further contains L-leucine.

According to another preferred embodiment of the present invention, the ratio of colloidal silicone dioxide to the total of L-leucine and sodium stearyl fumarate is between 10:1 to 1:60.

According to a further preferred embodiment of the present invention, the amount of sucralose is 8 to 15% by weight of the total weight of the composition.

According to another preferred embodiment of the present invention, said formulation disintegrates in a liquid with a temperature of 70-80°C in 60 seconds. According to a further preferred embodiment of the present invention, said formulation disintegrates in a liquid with a temperature of 70-80°C in 30 seconds. According to another preferred embodiment of the present invention, said filler or filling agent is at least one of mannitol, erythritol, or a proper mixture thereof. Said filler is preferably mannitol.

According to a further preferred embodiment of the present invention, a tablet is compressed at a pressure of 13 - 20 N.

According to another preferred embodiment of the present invention, said formulation is in the form of tablet, granule or powder.

According to a further preferred embodiment of the present invention, the sweetener formulation consisting of
a. sucralose at 8 to 15%
b. D-mannitol at 70 to 85%
c. croscarmellose sodium at 2 to 5%
d. colloidal silicone dioxide at 0.1 to 2%
e. L-leucine at 7 to 13%
f. sodium stearyl fumarate at 0.1 to 5%

According to another preferred embodiment of the present invention, said sweetener formulation is produced by a method, comprising the steps of;
a. adding mannitol, L-leucine, and croscarmellose sodium to sucralose, and mixing these ingredients,
b. adding a mixture of colloidal silicone dioxide and sodium stearyl fumarate to the first mixture obtained above, and mixing them together,
c. compressing this mixture in the form of tables.

### Detailed Description of Invention

### Example:

| **Unit Formula** | **Amount (mg)** | **Function** |
|---|---|---|
| Sucralose | 4.95 | Sweetener |
| D-Mannitol | 33.43 | Filler |
| Croscarmellose sodium | 1.44 | Disintegrant |
| Colloidal silicone dioxide | 0.12 | Glidant |
| L-Leucine | 4.50 | Lubricant |
| Sodium stearyl fumarate | 0.56 | Lubricant |
| Total | 45.00 | |

The sweetener formulation given in this example is produced by adding to sucralose; mannitol, L-leucine, and croscarmellose sodium, mixing these ingredients, adding a mixture of colloidal silicone dioxide and sodium stearyl fumarate to this first mixture and mixing them together, and compressing the resulting mixture in the form of tablets. The production preferably involves direct compressing method. It can alternatively be produced by wet granulation. In this formulation, the sweetener sucralose, the mannitol used as a filler, and other excipients do not carry any glycemic index potential. Thus, a product is obtained which can be used by those who are in diets or by the diabetics.

Sodium stearyl fumarate is a very efficient lubricant, being less hydrophobic than magnesium stearate and having a lower delaying effect, as compared to magnesium stearate, when the tablet is dissolved. Sodium stearyl fumarate neither gives rise to overmixing-related problems, which is observed with magnesium stearate. (Handbook Pharmaceutical Excipients, 5. Edition, Rowe, Raymond C., Sheskey, Paul J., Owen, Sian C., pp. 705-707).

The present invention involves the use of sodium stearyl fumarate and L-leucine (the lubricant) and colloidal silicone dioxide (the glidant) in the form of a combination, thereby eliminating the problems related to decreasing flowability and to the sticking of these materials to the production apparatuses and elements.

Additionally, the product's dissolution rate is brought to a very satisfactory extent, thanks to using croscarmellose sodium as a disintegrant.

Suitable fillers include, but are not restricted to microcrystalline cellulose, starch, mannitol, erythritol, or the mixtures thereof. The most preferred filler is mannitol.

In conclusion, the preferred embodiment described above provides a sweetener composition, which does not raise the glycemic index and provides the advantage of production simplicity.

## Claims

1. A sweetener formulation, **characterized in that** it comprises,
- sucralose, as high intensity sweetener, at 1-15%,
- croscarmellose sodium and colloidal silicone dioxide at a weight ratio between 20:1 and 1:10 (croscarmellose sodium : colloidal silicone dioxide),
- sodium stearyl fumarate, and
- at least one filler.

2. The sweetener formulation according to Claim 1, further comprising L-leucine.

3. The sweetener formulation according to any of the preceding claims, wherein the ratio of colloidal silicone dioxide to the total of L-leucine and sodium stearyl fumarate is between 10:1 and 1:60.

4. The sweetener formulation according to any of the preceding claims, **characterized in that** the amount of sucralose is 8 to 15% by weight of total composition.

5. The sweetener formulation according to any of the preceding claims, **characterized in that** said formulation disintegrates in a liquid at a temperature of 70-80°C in 60 seconds.

6. The sweetener formulation according to any of the preceding claims, **characterized in that** said formulation disintegrates in a liquid at a temperature of 70-80°C in 30 seconds.

7. The sweetener formulation according to any of the preceding claims, **characterized in that** said filler is at least one of mannitol, erythritol, or a mixture thereof.

8. The sweetener formulation according to claims 1 and 8, wherein said filler is preferably mannitol.

9. The sweetener formulation according to any of the preceding claims, wherein said formulation is in the form of a tablet, granule or powder.

10. The sweetener formulation according to any of the preceding claims, wherein said tablet is compressed at a pressure of 13 to 20 N.

11. The sweetener formulation according to any of the preceding claims, consisting of:
a. sucralose at 8 to 15%
b. D-mannitol at 70 to 85%
c. croscarmellose sodium at 2 to 5%
d. colloidal silicone dioxide at 0.1 to 2%
e. L-leucine at 7 to 13%
f. sodium stearyl fumarate at 0.1 to 5%.

12. A method for preparing a sweetener formulation according to any of the preceding claims, comprising the steps of:
a. adding mannitol, L-leucine, and croscarmellose sodium to sucralose, and mixing these ingredients,
b. adding a mixture of colloidal silicone dioxide and sodium stearyl fumarate to the first mixture obtained above, and mixing them together,
c. compressing the resulting mixture into tablets.

## Patentansprüche

1. Süßstoff-Formulierung, **dadurch gekennzeichnet, dass** sie umfasst:
- 1-15% Sucralose, als Intensiv-Süßstoff,
- Croscarmellose-Natrium und kolloidales Siliziumdioxid in einem Gewichtsverhältnis von zwischen 20:1 und 1:10 (Croscarmellose-Natrium : kolloidales Siliziumdioxid),
- Natriumstearylfumarat und
- mindestens einen Füllstoff.

2. Süßstoff-Formulierung nach Anspruch 1, ferner umfassend L-Leucin.

3. Süßstoff-Formulierung nach einem der vorangehenden Ansprüche, wobei das Verhältnis von kolloidalem Siliziumdioxid zur Gesamtmenge an L-Leucin und Natriumstearylfumarat zwischen 10:1 und 1:60 beträgt.

4. Süßstoff-Formulierung nach einem der vorangehenden Ansprüche, **dadurch gekennzeichnet, dass** die Menge an Sucralose 8 bis 15 Gewichts-% der Gesamtzusammensetzung beträgt.

5. Süßstoff-Formulierung nach einem der vorangehenden Ansprüche, **dadurch gekennzeichnet, dass** die Formulierung in einer Flüssigkeit bei einer Temperatur von 70-80°C in 60 Sekunden zerfällt.

6. Süßstoff-Formulierung nach einem der vorangehenden Ansprüche, **dadurch gekennzeichnet, dass** die Formulierung in einer Flüssigkeit bei einer Temperatur von 70-80°C in 30 Sekunden zerfällt.

7. Süßstoff-Formulierung nach einem der vorangehenden Ansprüche, **dadurch gekennzeichnet, dass** der Füllstoff mindestens eines von Mannitol, Erythritol oder einer Mischung davon ist.

8. Süßstoff-Formulierung nach den Ansprüchen 1 und 8, wobei der Füllstoff vorzugsweise Mannitol ist.

9. Süßstoff-Formulierung nach einem der vorangehenden Ansprüche, wobei die Formulierung in Form einer Tablette, eines Granulats oder eines Pulvers ist.

10. Süßstoff-Formulierung nach einem der vorangehenden Ansprüche, wobei die Tablette bei einem Druck von 13 bis 20 N gepresst ist.

11. Süßstoff-Formulierung nach einem der vorangehenden Ansprüche, bestehend aus:
a. 8 bis 15% Sucralose
b. 70 bis 85% D-Mannitol
c. 2 bis 5% Croscarmellose-Natrium
d. 0,1 bis 2% kolloidalem Siliciumdioxid
e. 7 bis 13% L-Leucin
f. 0,1 bis 5% Natriumstearylfumarat.

12. Methode zur Herstellung einer Süßstoff-Formulierung nach einem der vorangehenden Ansprüche, umfassend die Schritte:
a. Hinzufügen von Mannitol, L-Leucin und Croscarmellose-Natrium zu Sucralose und Mischen dieser Bestandteile,
b. Hinzufügen einer Mischung von kolloidalem Siliciumdioxid und Natriumstearylfumarat zur ersten oben erhaltenen Mischung und Vermischen derselben
c. Verpressen der resultierenden Mischung in Tabletten.

## Revendications

1. Formulation d'édulcorant, **caractérisée en ce qu'**elle comprend,
- du sucralose, en tant qu'édulcorant de haute intensité, à hauteur de 1-15 %,
- de la croscarmellose sodique et du dioxyde de silicium colloïdal selon un rapport pondéral compris entre 20/1 et 1/10 (croscarmellose sodique/silice colloïdale),
- du stéarylfumarate de sodium, et
- au moins un agent de remplissage.

2. Formulation d'édulcorant selon la revendication 1, comprenant en outre de la L-leucine.

3. Formulation d'édulcorant selon l'une quelconque des revendications précédentes, dans laquelle le rapport entre le dioxyde de silicium colloïdal et le total de la L-leucine et du stéarylfumarate de sodium se situe entre 10/1 et 1/60.

4. Formulation d'édulcorant selon l'une quelconque des revendications précédentes, **caractérisée en ce que** la quantité de sucralose est de 8 à 15 % en poids de la composition totale.

5. Formulation d'édulcorant selon l'une quelconque des revendications précédentes, **caractérisée en ce que** ladite formulation se désintègre dans un liquide à une température de 70-80°C en 60 secondes.

6. Formulation d'édulcorant selon l'une quelconque des revendications précédentes, **caractérisée en ce que** ladite formulation se désintègre dans un liquide à une température de 70-80°C en 30 secondes.

7. Formulation d'édulcorant selon l'une quelconque des revendications précédentes, **caractérisée en ce que** ledit agent de remplissage est au moins l'un parmi le mannitol, l'érythritol, ou un mélange de ceux-ci.

8. Formulation d'édulcorant selon les revendications 1 et 8, dans laquelle ledit agent de remplissage est de préférence le mannitol.

9. Formulation d'édulcorant selon l'une quelconque des revendications précédentes, dans laquelle ladite formulation se présente sous la forme d'un comprimé, d'un granulé ou d'une poudre.

10. Formulation d'édulcorant selon l'une quelconque des revendications précédentes, dans laquelle ledit comprimé est aggloméré par compression à une pression de 13 à 20 N.

11. Formulation d'édulcorant selon l'une quelconque des revendications précédentes, constituée par :
a. du sucralose à hauteur de 8 à 15 %
b. du D-mannitol à hauteur de 70 à 85 %
c. de la croscarmellose sodique à hauteur de 2 à 5 %
d. du dioxyde de silicium colloïdal à hauteur de 0,1 à 2 %
e. de la L-leucine à hauteur de 7 à 13 %
f. du stéarylfumarate de sodium à hauteur de 0,1 à 5 %.

12. Procédé de préparation d'une formulation d'édulcorant selon l'une quelconque des revendications précédentes, comprenant les étapes consistant à :
a. ajouter le mannitol, la L-leucine et la croscarmellose sodique au sucralose, et mélanger ces ingrédients,
b. ajouter un mélange de dioxyde de silicium colloïdal et de stéarylfumarate de sodium au premier mélange obtenu ci-dessus et les mélanger ensemble,
c. agglomérer par compression le mélange résultant pour obtenir des comprimés.
